(19) Europäisches Patentamt
European Patent Office
Office européen des brevets

(11) **EP 1 129 695 A1**

(12) **EUROPÄISCHE PATENTANMELDUNG**

(43) Veröffentlichungstag:
**05.09.2001 Patentblatt 2001/36**

(51) Int Cl.⁷: **A61K 7/42**

(21) Anmeldenummer: **01101426.3**

(22) Anmeldetag: **23.01.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **29.02.2000 DE 10009442**
**07.09.2000 DE 10044351**

(71) Anmelder: **BASF AKTIENGESELLSCHAFT
67056 Ludwigshafen (DE)**

(72) Erfinder:
- **Heidenfelder, Thomas, Dr.
67354 Römerberg (DE)**
- **Schehlmann Volker, Dr.
Rockaway TWP,NJ 07866 (US)**
- **Wünsch, Thomas, Dr.
67346 Speyer (DE)**
- **Dausch, Wilma M., Dr.
67117 Limburgerhof (DE)**

(54) **Kosmestische oder dermatologische Lichtschutzmittelzubereitungen**

(57) Die Erfindung betrifft kosmetische oder dermatologische Zubereitungen, enthaltend

a) 0,1 bis 10 Gew.-% eines oder mehrerer 3,3-Diphenylacrylate der Formel I,

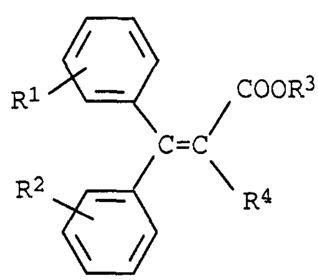

I

und

b) 0,1 bis 10 Gew.-% eines oder mehrerer Dibenzoylmethanderivate der Formel II,

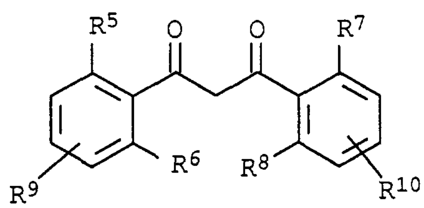

II

in der die Substituenten $R^1$ bis $R^{10}$ unabhängig voneinander die in der Beschreibung genannte Bedeutung haben.

EP 1 129 695 A1

## Beschreibung

Die Erfindung betrifft kosmetische oder dermatologische Lichtschutzmittelzubereitungen, enthaltend eine Kombination aus einem oder mehreren 3,3-Diphenylacrylaten und einem oder mehreren Dibenzoylmethanderivaten sowie deren Verwendung zum Schutz der menschlichen Haut oder menschlicher Haare gegen UV-Strahlen.

Die in kosmetischen und dermatologischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich u.a. um Derivate des 3-Benzylidencamphers, der 4-Aminobenzoesäure, der Zimtsäure, der Salicylsäure, des Benzophenons sowie des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen UVA-Strahlen werden Derivate des Dibenzoylmethans verwendet, deren Photostabilität jedoch nicht in ausreichendem Maße gegeben ist (Int. J. Cosm. Science 10, 53 (1988)).

In der französischen Patentschrift 2,440,933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß US 5,587,150 und US 5,576,354 die nicht ausreichend photostabilen UV-A-Filter durch den Zusatz von 2-Cyan-3,3-diphenylacrylsäureestern stabilisiert werden, die selbst im UV-B-Bereich als Filter dienen.

Die in US 5,587,150 und US 5,576,354 genannten Kombinationen aus Dibenzoylmethan und 2-Cyan-3,3-diphenylacrylsäureester haben den Nachteil, daß die damit hergestellten Zubereitungen in vielen Fällen noch unzureichende kosmetische Eigenschaften aufweisen, wie z.B. zu hohe Klebrigkeit und damit verbunden ein unbefriedigendes Hautgefühl.

Es bestand daher die Aufgabe, neue Stabilisatoren für UV-A-Filter aus der Klasse der Dibenzoylmethane bereitzustellen, die die o.g. Nachteile nicht aufweisen.

Ferner war es Aufgabe, Lichtschutzmittel für kosmetische und dermatologische Zwecke vorzuschlagen, die im UV-A-Bereich mit hoher Extinktion absorbieren, die photostabil sind, eine geringe Eigenfarbe d.h. eine scharfe Bandenstrukur aufweisen und in Öl oder Wasser gut verarbeitbar sind.

Diese Aufgabe wurde gelöst durch kosmetische oder dermatologische Zubereitungen, enthaltend

a) 0,1 bis 10 Gew.-% eines oder mehrerer 3,3-Diphenylacrylate der Formel I,

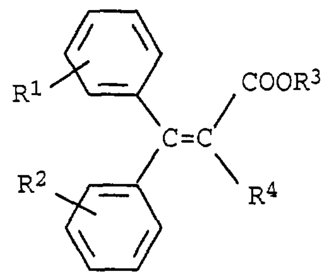

I

in der die Substituenten unabhängig voneinander folgende Bedeutung haben:

$R^1$ und $R^2$ Wasserstoff, $C_1$-$C_{12}$-Alkyl, $C_1$-$C_{12}$-Alkoxy;

$R^3$ $C_3$-$C_{10}$-Cycloalkyl, gegebenenfalls substituiert;

$R^4$ Wasserstoff, CN

und

b) 0,1 bis 10 Gew.-% eines oder mehrerer Dibenzoylmethanderivate der Formel II,

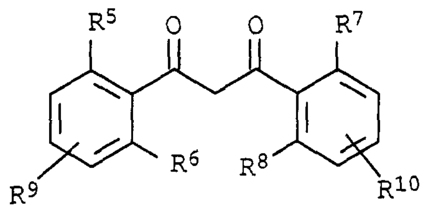

II

in der die Substituenten unabhängig voneinander folgende Bedeutung haben:

$R^5$ bis $R^8$ Wasserstoff $C_1$-$C_4$-Alkyl:

$R^9$ und $R^{10}$ Wasserstoff, $C_1$-$C_{12}$-Alkyl, $C_1$-$C_{12}$-Alkoxy.

Als Alkylreste für $R^1$ und $R^2$ sowie $R^9$ und $R^{10}$ seien verzweigte oder unverzweigte $C_1$-$C_{12}$-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, 1,1,3-Trimethylhexyl, 1,1,3,3-Tetramethylpentyl, n-Nonyl, n-Decyl, n-Undecyl, 1-Methylundecyl, n-Dodecyl genannt.
Als Alkylreste für $R^5$ bis $R^8$ seien verzweigte oder unverzweigte $C_1$-$C_4$-Alkylketten, bevorzugt Methyl, Ethyl, n-Propyl, 1-Methylethyl, n-Butyl, 1-Methylpropyl-, 2-Methylpropyl, 1,1-Dimethylethyl genannt.
Als Alkoxyreste für $R^1$ und $R^2$ sowie $R^9$ und $R^{10}$ kommen gradkettige und verzweigte Reste mit 1 bis 12 C-Atomen, bevorzugt mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| 1-Methyl-1-ethylpropoxy- | Heptoxy- |
| Octoxy- | 2-Ethylhexoxy- |

Als Cycloalkylreste seien für $R^3$ $C_3$-$C_{10}$-Cycloalkylreste wie Cyclopropyl, Cyclobutyl, Cyclopentyl, Cyclohexyl, Cycloheptyl, Cyclooctyl genannt. Bevorzugt sind $C_5$-$C_8$-Cycloalkyl wie Cyclopentyl, Cyclohexyl, Cycloheptyl und Cyclooctyl, insbesondere Cyclopentyl und Cyclohexyl.

Die Cycloalkylreste können ggf. mit bis zu drei Resten u.a. Halogen z.B. Fluor, Chlor oder Brom, Cyano, Nitro, Amino, $C_1$-$C_4$-Alkylamino, $C_1$-$C_4$-Dialkylamino, Hydroxy, $C_1$-$C_4$-Alkyl oder $C_1$-$C_4$-Alkoxy substituiert sein oder 1 bis 3 Heteroatome wie Schwefel, Stickstoff, dessen freie Valenzen durch Wasserstoff oder $C_1$-$C_4$-Alkyl abgesättigt sein können oder Sauerstoff im Ring enthalten.

Die Cycloalkylreste können auch als bicyclische Ringsysteme vorliegen.

Bevorzugte Substituenten am Cycloalkylring sind $C_1$-$C_4$-Alkylgruppen, insbesondere Methyl, Ethyl, n-Propyl, 1-Methylethyl, n-Butyl, 1-Methylpropyl-, 2-Methylpropyl, 1,1-Dimethylethyl.

Als besonders bevorzugte Substituenten für $R^3$ sind die im folgenden aufgeführten Cycloalkylreste zu nennen.

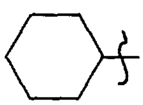 , 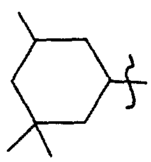 , 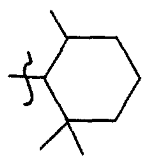 , 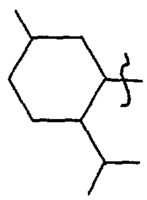 , 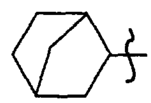 , 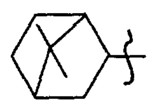

Bevorzugt sind kosmetische oder dermatologische Zubereitungen, enthaltend

a) 0,1 bis 10 Gew.-% eines oder mehrerer 3,3-Diphenylacrylate der Formel Ib,

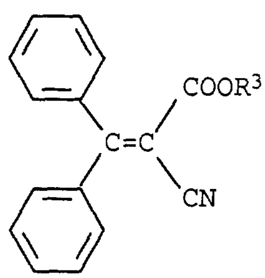

Ib

in der $R^3$ für $C_5$-$C_8$-Cycloalkyl, gegebenenfalls substituiert steht
und

b) 0,1 bis 10 Gew.-% eines oder mehrerer Dibenzoylmethanderivate, ausgewählt aus der Gruppe, bestehend aus 2-Methyldibenzoylmethan, 4-Methyldibenzoylmethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4-tert.-Butyl-4'-methoxydibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan, 2,6-Dimethyl-4-tert.-butyl-4'-methoxydibenzoylmethan.

Besonders bevorzugt sind kosmetische oder dermatologische Zubereitungen, enthaltend

a) 0,1 bis 10 Gew.-% eines oder mehrerer 3,3-Diphenylacrylate der Formel Ib,

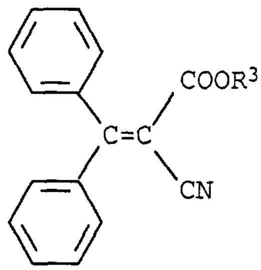

Ib

in der $R^3$ für $C_5$-$C_8$-Cycloalkyl, gegebenenfalls substituiert steht und

b) 0,1 bis 10 Gew.-% 4-tert.-Butyl-4'-methoxydibenzoylmethan.

Der Gehalt an Komponente a) in den erfindungsgemäßen kosmetischen oder dermatologischen Lichtschutzmittelzubereitungen liegt im Bereich von 0,1 bis 10 Gew.-%, bevorzugt 0,1 bis 6 Gew.-%, besonders bevorzugt im Bereich von 0,3 bis 3 Gew.-%, ganz besonders bevorzugt im Bereich von 1,5 bis 2,5 Gew.-%.

Der Gehalt an Komponente b) in den erfindungsgemäßen kosmetischen oder dermatologischen Lichtschutzmittelzubereitungen liegt im Bereich von 0,1 bis 10 Gew.-%, bevorzugt 0,2 bis 7 Gew.-%, besonders bevorzugt im Bereich von 0,3 bis 6 Gew.-%, ganz besonders bevorzugt im Bereich von 0,4 bis 5 Gew.-%.

Das molare Verhältnis der Komponenten a) und b) in den kosmetischen oder dermatologischen Zubereitungen liegt in einem Bereich a:b von 0,5 bis 10, bevorzugt in einem Bereich von 1 bis 8.

Die erfindungsgemäßen kosmetischen oder dermatologischen Zubereitungen zeigen einen Lichtschutzfaktor größer 5, bevorzugt größer 10, besonders bevorzugt größer 20, ganz besonders bevorzugt größer 25.

Die Lichtschutzmittel enthaltenden kosmetischen und dermatologischen Zubereitungen sind in der Regel auf der Basis eines Trägers, der mindestens eine Ölphase enthält. Demgemäß kommen Öle, Öl-in-Wasser- und Wasser-in-Öl-Emulsionen, Cremes und Pasten, Lippenschutzstiftmassen oder fettfreie Gele in Betracht.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $C3_2O_3$), Mischoxiden der entsprechenden Metalle sowie Abmischungen aus solchen Oxiden. Besonders bevorzugt handelt es sich um Pigmente auf Basis von $TiO_2$.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$nTiO_2 + m(RO)_3Si\text{-}R' \rightarrow nTiO_2 \text{ (oberfl.)}$$

erzeugt wird, n und m sind dabei nach Belieben einzusetzende stöchiometrische Parameter, R und R' die gewünschten organischen Reste. Beispielsweise sind hydrophobisierte Pigmente von Vorteil, die in Analogie zu DE-OS 33 14 742

dargestellt wurden.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die kosmetischen oder dermatologischen Lichtschutzzubereitungen enthalten vorteilhaft anorganische Pigmente, insbesondere Mikropigmente, in Mengen von z.B. 0,1 Gew.-% bis 30 Gew.-%, bevorzugt in Mengen von 0,5 Gew.-% bis 15 Gew.-%, besonders bevorzugt 1 Gew.-% bis 10 Gew.-%, ganz besonders bevorzugt 1,5 bis 6 Gew.-%, bezogen auf das Gesamtgewicht der Zubereitungen.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für die Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UV-A-Filter und/oder mindestens einen weiteren UV-B-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der oben genannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen. Sofern Vitamin A bzw. Vitamin-A-Derivate bzw. Carotine bzw. deren Derivate das oder die Antixoidantien darstellen, ist es vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;

- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/ oder ungesättigten, verzweigten und/oder unverzweigten Alkancarbonsäuren einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstereat, Isopropyloleat, n-Butylstereat, N-Hexyllaurat, N-Decyloleat, Isooctylstearat, Isononylstereat, Isononylisnoanoal, 2-Ethylhexylpalmitat, 2-Ethylhexyllaurat, 2-Hexyldecylstereat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisonnanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -mono-butylether, Propylenglykolmonomethyl, -monoethyl- oder -mono-butylether, Diethylenglykolmonomethyl- oder -monoethylether und analoge Produkte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Es ist weiterhin vorteilhaft, außer den erfindungsgemäßen Kombinationen zusätzliche öllösliche organische UV-A-Filter und/oder UV-B-Filter in der Lipidphase und/oder wasserlösliche organische UV-A-Filter und/oder UV-B-Filter in der wäßrigen Phase einzusetzen, wobei die Gesamtmenge der Filtersubstanzen z.B. 0,1 Gew.-% bis 30 Gew.-%, vorzugsweise 0,5 bis 15 Gew.-%, insbesondere 1 bis 10 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Beispielsweise sind zu nennen:

Tabelle 1:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | Menthyl-o-aminobenzoate oder: 5-Methyl-2-(1-methylethyl)-2-aminobenzoate | 134-09-8 |
| 20 | Glyceryl p-aminobenzoat oder: 4-Aminobenzoesäure-1-glyceryl-ester | 136-44-7 |
| 21 | 2,2'-Dihydroxy-4-methoxybenzophenon (Dioxybenzone) | 131-53-3 |
| 22 | 2-Hydroxy-4-methoxy-4-methylbenzophenon (Mexenon) | 1641-17-4 |
| 23 | Triethanolamin Salicylat | 2174-16-5 |
| 24 | Dimethoxyphenylglyoxalsäure oder: 3,4-dimethoxy-phenyl-glyoxal-saures Natrium | 4732-70-1 |
| 25 | 3-(4'Sulfo)benzyliden-bornan-2-on und seine Salze | 56039-58-8 |
| 26 | 2,2',4,4'-Tetrahydroxybenzophenon | 131-55-5 |
| 27 | 2,2'-Methylen-bis-[6(2H-benzotriazol-2-yl)-4-(1,1,3,3,-tetramethylbutyl)phenol] | 103597-45-1 |
| 28 | 2,2'-(1,4-Phenylen)-bis-lH-benzimidazol-4,6-disulfonsäure, Na-Salz | 180898-37-7 |
| 29 | 2,4-bis-[4-(2-Ethylhexyloxy)-2-hydroxy]phenyl-6-(4-methoxyphenyl)-(1,3,5)-triazin | 187393-00-6 |
| 30 | 3-(4-Methylbenzyliden)-campher | 36861-47-9 |
| 31 | 4-Bis(polyethoxy)paraaminobenzoesäurepolyethoxyethylester | 113010-52-9 |
| 32 | 2,4-Dihydroxybenzophenon | 131-56-6 |
| 33 | 2,2'-Dihydroxy-4,4'-dimethoxybenzophenon-5,5'-dinatriumsulfonat | 3121-60-6 |

Weitere kombinierbare Lichtschutzmittel sind u.a. folgende Verbindungen:

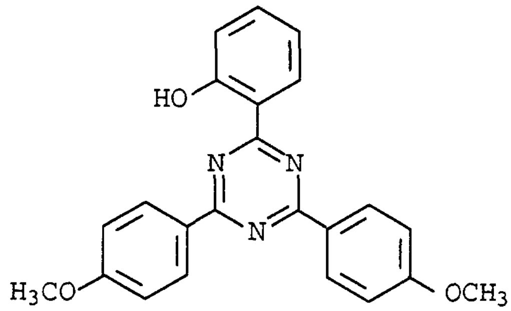

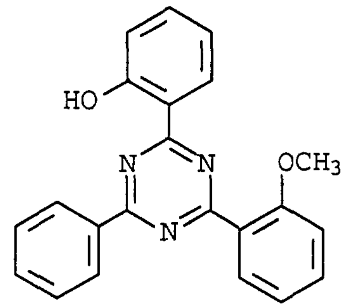

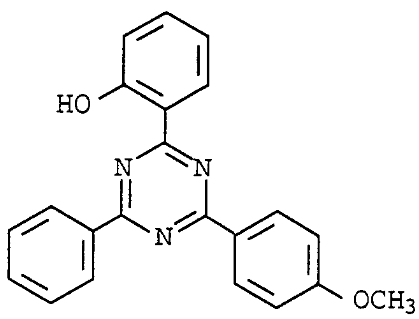

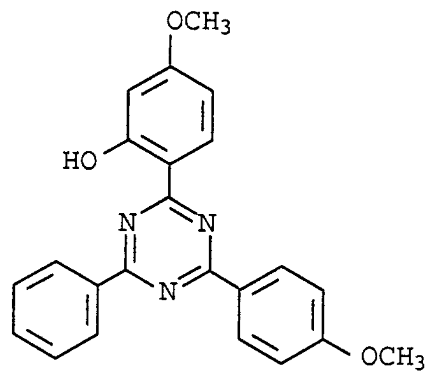

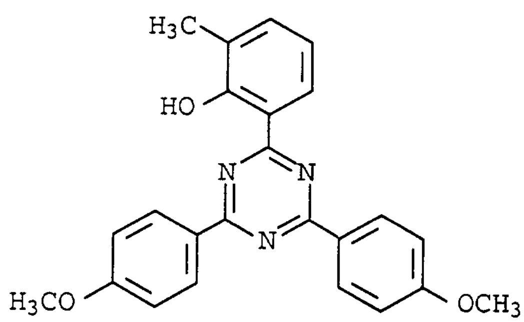

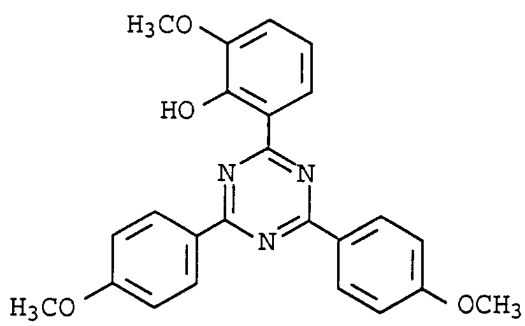

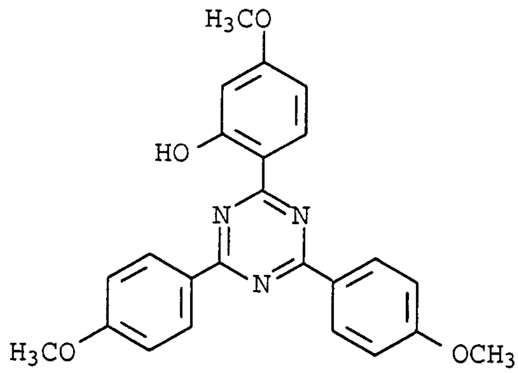

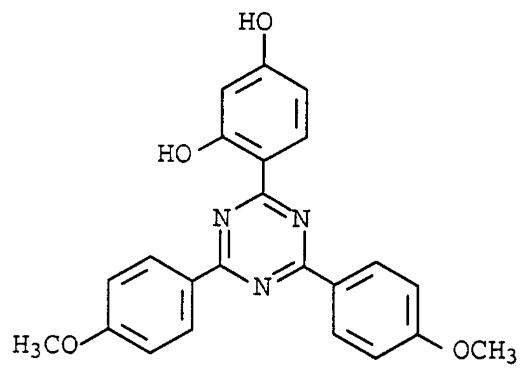

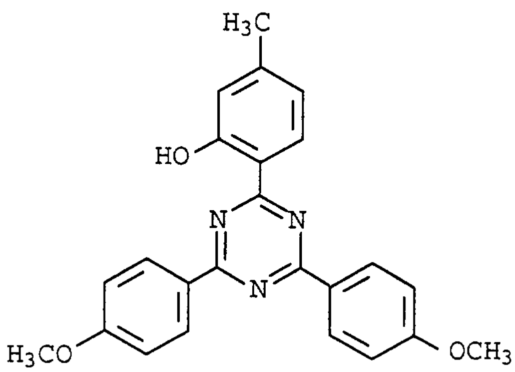

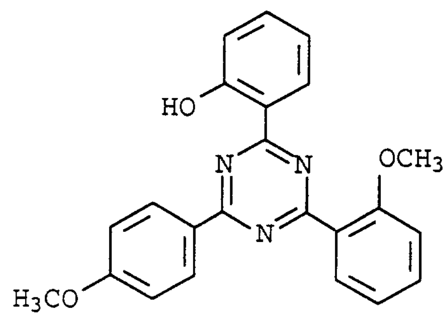

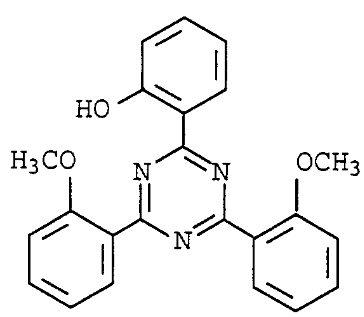

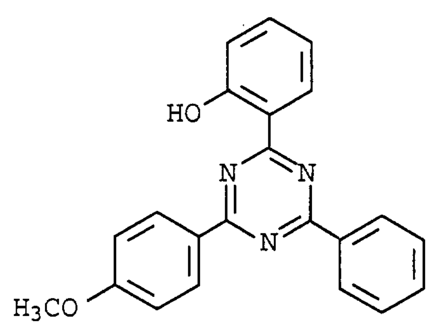

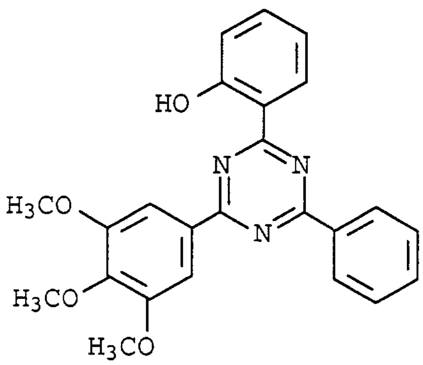

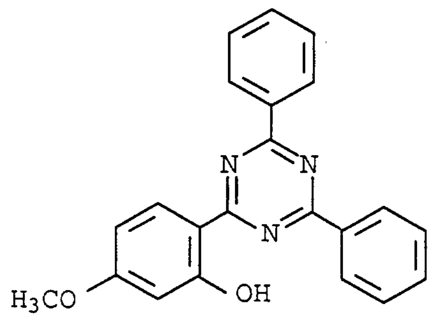

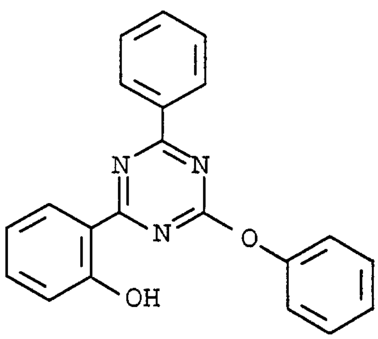

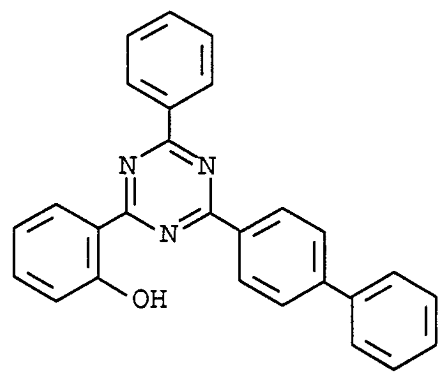

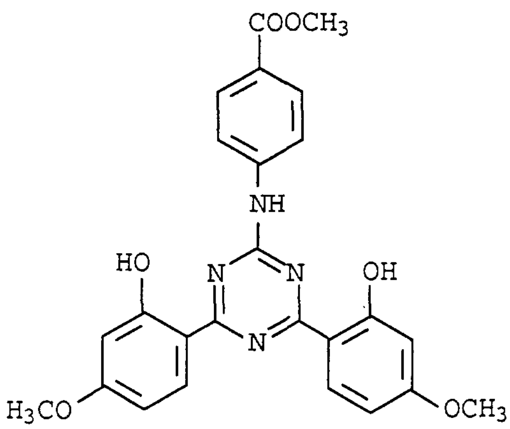

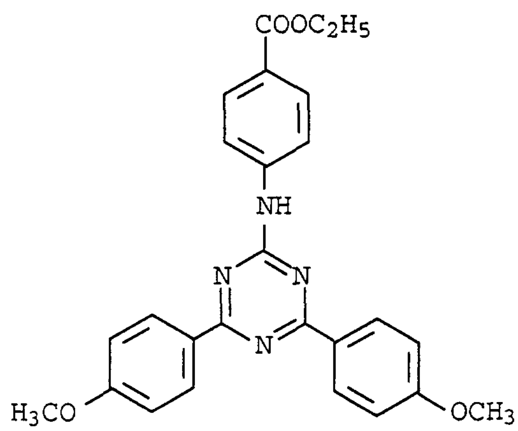

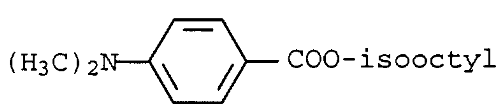

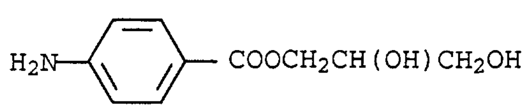

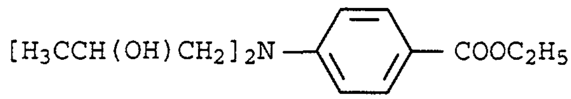

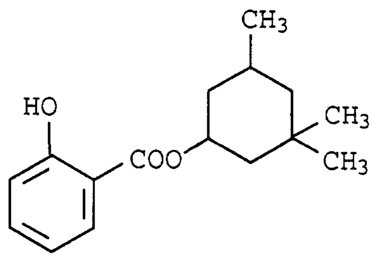

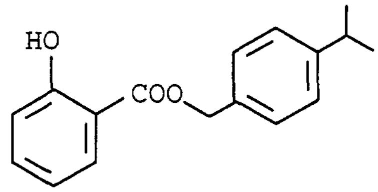

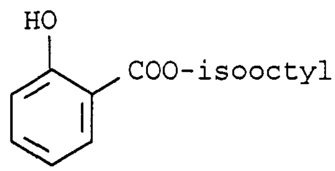

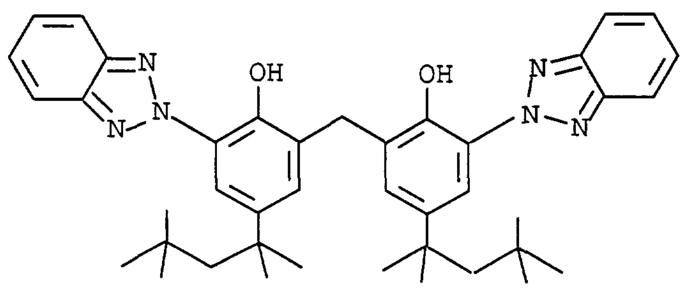

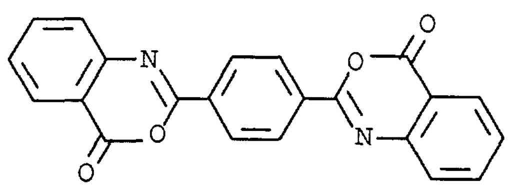

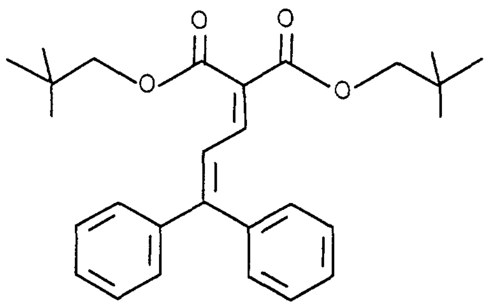 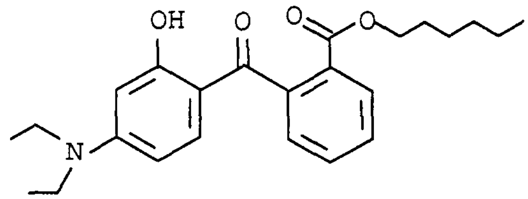

Die Liste der genannten UV-Filter, die in Kombination mit den erfindungsgemäßen Wirkstoffkombinationen verwendet werden können, soll selbstverständlich nicht limitierend sein.

Zum Schutz menschlicher Haare vor UV-Strahlen können die erfindungsgemäßen Lichtschutzzubereitungen als Shampoos, Lotionen, Gelen, Haarsprays, Aerosol-Schaumcremes oder Emulsionen u.a. zum Waschen, Färben sowie zum Frisieren der Haare verwendet werden.

Die UV-Filterwirkung der erfindungsgemäßen Zubereitungen kann auch zur Stabilisierung von Wirk- und Hilfsstoffen in kosmetischen und dermatologischen Formulierungen ausgenutzt werden.

Die erfindungsgemäße Kombination zeichnet sich insbesondere dadurch aus, daß sie zu einer deutlichen Photostabilisierung von Dibenzoylmethanderivaten führt (siehe Beispiel 14).

Die folgenden Beispiele sollen die vorliegende Erfindung verdeutlichen, ohne sie einzuschränken.

I. Herstellung

Beispiel 1: 2-Cyan-3,3-diphenylacrylsäure-cyclopentylester

83,1 g (0,3 mol) 2-Cyan-3,3-diphenylacrylsäureethylester (Uvinul 3035), 100 ml Cyclopentanol und 6 g $Na_2CO_3$ wurden bei 145 °C unter destillativer Entfernung des gebildeten Ethanols, die durch einen Stickstoffstrom unterstützt wurde, miteinander umgesetzt. Nach etwa 3 h wurde das Reaktionsgemisch heiß filtriert, um das $Na_2CO_3$ zu entfernen. Nach dem Abkühlen des Filtrats wurde der ausgefallene Niederschlag abfiltriert, mit Petrolether gewaschen und getrocknet, wobei 78,1 g (82%) 2-Cyan-3,3-diphenylacrylsäure-cyclopentylester als farbloser Feststoff erhalten wurden. [$\lambda_{max}$ 300 nm; $E^1_1$ (MeOH) 395]

Beispiel 2: 2-Cyan-3,3-diphenylacrylsäure-cyclohexylester

139 g (0,5 mol) 2-Cyan-3,3-diphenylacrylsäureethylester (Uvinul 3035), 200 ml Cyclohexanol und 10 g $Na_2CO_3$ wurden bei 150 °C unter destillativer Entfernung des gebildeten Ethanols, die durch einen Stickstoffstrom unterstützt wurde, miteinander umgesetzt. Nach etwa 6 h wurde das Reaktionsgemisch heiß filtriert, um das $Na_2CO_3$ zu entfernen. Nach dem Abkühlen des Filtrats wurde der ausgefallene Niederschlag abfiltriert, mit Petrolether gewaschen und getrocknet, wobei 111 g (67%) 2-Cyan-3,3-diphenylacrylsäure-cyclohexylester als farbloser Feststoff erhalten wurden. [$\lambda_{max}$ 300 nm; $E^1_1$ (MeOH) 381]

Beispiel 3: 2-Cyan-3,3-diphenylacrylsäure-4'-tert.-butyl-cyclohexylester

139 g (0,5 mol) 2-Cyan-3,3-diphenylacrylsäureethylester (Uvinul 3035), 200 ml 4-*tert.*-Butylcyclohexanol und 10 g $Na_2CO_3$ wurden bei 182 °C unter destillativer Entfernung des gebildeten Ethanols, die durch einen Stickstoffstrom unterstützt wurde, miteinander umgesetzt. Nach etwa 4 h wurde das Reaktionsgemisch mit 300 ml Methanol verdünnt und heiß filtriert, um das $Na_2CO_3$ zu entfernen. Nach dem Abkühlen des Filtrats wurde der ausgefallene Niederschlag abfiltriert, mit Petrolether gewaschen und getrocknet, wobei 130 g (67%) 2-Cyan-3,3-diphenylacrylsäure-4'-tert.-butylcyclohexylesterals farbloser Feststoff erhalten wurden. [$\lambda_{max}$ 302 nm; $E^1_1$ (MeOH) 324]

Beispiel 4: 2-Cyan-3,3-diphenylacrylsäure-norborneylester

55,4 g (0,2 mol) 2-Cyan-3,3-diphenylacrylsäureethylester (Uvinul 3035), 100 g Norborneol und 4 g $Na_2CO_3$ wurden bei ca. 185 °C unter destillativer Entfernung des gebildeten Ethanols, die durch einen Stickstoffstrom unterstützt wurde, miteinander umgesetzt. Nach etwa 4 h wurde das Reaktionsgemisch heiß filtriert, um das $Na_2CO_3$ zu entfernen.

Nach dem Abkühlen des Filtrats wurde der ausgefallene Niederschlag abfiltriert, mit Cyclohexan gewaschen und getrocknet, wobei 37 g (53%) 2-Cyan-3,3-diphenylacrylsäure-norborneylester [$\lambda_{max}$=304 nm, $E^1_1$ = 386] als farbloser Feststoff erhalten wurden.

Beispiel 5: 2-Cyan-3,3-diphenylacrylsäure-2',2',6'-trimethyl-cyclohexylester

55,4 g (0,2 mol) 2-Cyan-3,3-diphenylacrylsäureethylester (Uvinul 3035), 100 ml 2,2,6-Trimethylcyclohexanol und 4 g $Na_2CO_3$ wurden bei 184-186 °C unter destillativer Entfernung des gebildeten Ethanols, die durch einen Stickstoffstrom unterstützt wurde, miteinander umgesetzt. Nach etwa 3 h wurde das Reaktionsgemisch heiß filtriert, um das $Na_2CO_3$ zu entfernen. Nach dem Abkühlen des Filtrats wurde im Vakuum destilliert, wobei 55 g (74%) 2-Cyan-3,3-diphenylacrylsäure-2',2',6'-trimethyl-cyclohexylester [$\lambda_{max}$=304 nm, $E^1_1$ = 351] als farbloser Feststoff erhalten wurden.

Beispiel 6: 2-Cyan-3,3-diphenylacrylsäure-3',3',5'-trimethyl-cyclohexylester

55,4 g (0,2 mol) 2-Cyan-3,3-diphenylacrylsäureethylester (Uvinul 3035), 100 ml 3,3,5-Trimethylcyclohexanol und 4 g $Na_2CO_3$ wurden bei 181-184 °C unter destillativer Entfernung des gebildeten Ethanols, die durch einen Stickstoffstrom unterstützt wurde, miteinander umgesetzt. Nach etwa 5 h wurde das Reaktionsgemisch heiß filtriert, um das $Na_2CO_3$ zu entfernen. Nach dem Abkühlen des Filtrats wurde der ausgefallene Niederschlag abfiltriert, mit Cyclohexan gewaschen und getrocknet, wobei 29 g (39%) 2-Cyan-3,3-diphenylacrylsäure-3',3',5'-trimethyl-cyclohexylesterder [$\lambda_{max}$=301 nm, $E^1_1$ = 344] als farbloser Feststoff erhalten wurden.

Beispiel 7: 2-Cyan-3,3-diphenylacrylsäure-menthylester

55,4 g (0,2 mol) 2-Cyan-3,3-diphenylacrylsäureethylester (Uvinul 3035), 62,5 g Menthol und 4 g $Na_2CO_3$ wurden bei 185-200 °C unter destillativer Entfernung des gebildeten Ethanols, die durch einen Stickstoffstrom unterstützt wurde, miteinander umgesetzt. Nach etwa 6 h wurde das Reaktionsgemisch abgekühlt und im Vakuum destilliert, wobei 44 g (57%) 2-Cyan-3,3-diphenylacrylsäure-menthylester [$\lambda_{max}$=300 nm, $E^1_1$ = 351] erhalten wurden.

II. Zubereitungen

Beispiel 8

Zusammensetzung für die Lippenpflege

| Massengehalt (Gew.-%) | |
|---|---|
| ad 100 | Eucerinum anhydricum |
| 10,00 | Glycerin |
| 10,00 | Titanium Dioxid, mikronisiert |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

Beispiel 9

Zusammensetzung für Sunblocker mit Mikropigmenten

| Massengehalt (Gew.-%) | |
|---|---|
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

Beispiel 10

Fettfreies Gel

| Massengehalt (Gew.-%) | |
|---|---|
| ad 100 | Wasser |
| 8,00 | Octyl Methoxycinnamat |
| 7,00 | Titanium Dioxid, mikronisiert |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

Beispiel 11

Sonnencreme (LSF 20)

| Massengehalt (Gew.-%) | |
|---|---|
| ad 100 | Wasser |

(fortgesetzt)

| Massengehalt (Gew.-%) | |
|---|---|
| 8,00 | Octyl Methoxycinnamat |
| 8,00 | Titanium Dioxid, mikronisiert |
| 6,00 | PEG-7-Hydrogenated Castor Öl |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

Beispiel 12

Sonnencreme wasserfest

| Massengehalt (Gew.-%) | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

Beispiel 13

Sonnenmilch (LSF 6)

| Massengehalt (Gew.-%) | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

Beispiel 14

Tageslotion mit UV-Schutz

| Massengehalt (Gew.-%) | |
|---|---|
| ad 100 | Wasser |
| 2,00 | Cetearyl Alkohol |
| 1,00 | Glycerinmonostearat |
| 2,00 | Vaselin |
| 7,50 | Octyl Methoxycinnamat |
| 4,00 | Octyl Salicylat |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 0,50 | Dimethicon |
| 5,00 | Propylen Glycol |
| 0,20 | EDTA |
| 0,20 | Carbomer |
| 5,00 | $C_{12}$-$C_{15}$ Alkyl Benzoat |
| 0,27 | Triethanolamin |
| 1,00 | Tocopheryl Acetat |
| q.s. | Fragrance |

Beispiel 15

Tagescreme mit UV-Schutz

| Massengehalt (Gew.-%) | |
|---|---|
| ad 100 | Wasser |
| 2,00 | Cetearyl Alkohol |

(fortgesetzt)

| Massengehalt (Gew.-%) | |
|---|---|
| 2,00 | Cetyl Alkohol |
| 1,00 | Glycerinmonostearat |
| 2,00 | Vaselin |
| 7,50 | Octyl Methoxycinnamat |
| 4,00 | Octyl Salicylat |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 4,00 | Propylen Glycol |
| 0,20 | EDTA |
| 0,20 | Carbomer |
| 0,20 | Xanthan |
| 0,20 | $C_{10}$-$C_{30}$ Alkyl Acrylate Crosspolymer |
| 5,00 | $C_{12}$-$C_{15}$ Alkyl Benzoat |
| 0,54 | Triethanolamin |
| 1,00 | Tocopheryl Acetat |
| q.s. | Fragrance |
| q.s. | Konservierungsmittel |

Beispiel 16

Flüssiges Make Up

| Massengehalt (Gew.-%) | |
|---|---|
| ad 100 | Wasser |
| 2,00 | Cetearyl Alkohol |
| 2,00 | Ceteareth 25 |
| 6,00 | Glycerinmonostearat |
| 1,00 | Cetylalkohol |
| 8,00 | Paraffinöl |
| 7,00 | Cetearyl Octanoat |
| 0,2 | Dimethicon |
| 3,00 | Propylen Glycol |
| 1,00 | Panthenol |
| 3,00 | 2-Cyan-3,3-diphenylacrylsäure-cyclohexylester (hergestellt gemäß Beispiel 2) |
| 1,50 | 4-tert.-Butyl-4'-methoxy-dibenzoylmethan |
| 3,50 | Octyl Methoxycinnamat |
| 0,1 | Bisabolol |
| 5,70 | Titandioxid |
| 1,10 | Eisenoxid |
| q.s. | Fragrance |

Beispiel 17

Haargel mit Sonnenschutz

| Massengehalt (Gew.-%) | |
|---|---|
| ad 100 | Wasser |
| 1,20 | Carbomer |
| 0,50 | Hydroxyethyl Cellulose |
| 4,00 | Triethanolamin |
| 0,70 | PEG-40 Hydrogenated Castor oil |
| 1,50 | 2-Cyan-3,3-diphenylacrylsäure-cyclohexylester (hergestellt gemäß Beispiel 2) |
| 0,70 | 4-tert.-Butyl-4'-methoxy-dibenzoylmethan |
| 2,80 | Octyl Methoxycinnamat |
| 5,00 | Propylen Glycol |
| 0,01 | EDTA |
| q.s. | Fragrance |
| q.s. | Sicovit Patentblau 85 E 131 |

Beispiel 18

Standardisierte Methode zur Bestimmung der Photostabilität (Suntest)

Eine 2 Gew.-%ige alkoholische Lösung des zu prüfenden Lichtschutzmittels wurde mittels einer Eppendorfpipette (20 µl) auf die Auffräsung eines Glasplättchens aufgetragen. Durch die Anwesenheit des Alkohols verteilte sich die Lösung gleichmäßig auf der aufgerauten Glasoberfläche. (Die aufgetragene Menge entspricht der Menge an Lichtschutzmittel, die in Sonnencremes zur Erreichung eines mittleren Lichtschutzfaktors benötigt wird.) Bei der Prüfung wurden jeweils 4 Glasplättchen bestrahlt. Die Bestrahlungszeit betrug je 30/60/90/120 Minuten. Die Glasplättchen wurden während des Bestrahlens durch eine Wasserkühlung, die sich am Boden des Suntestgeräte befindet, leicht gekühlt. Die Temperatur innerhalb des Suntest Gerätes betrug während der Bestrahlung 40°C. Nachdem die Proben bestrahlt worden waren, wurden sie mit Ethanol in einen dunklen 50 ml Meßkolben gewaschen und mit dem Photometer vermessen. Die Blindproben wurden ebenso auf Glasplättchen aufgetragen und 30 Minuten bei Raumtemperatur abgedampft. Wie die anderen Proben wurden sie mit Ethanol abgewaschen und auf 100 ml verdünnt und vermessen.

| Stabilisator | Photostabilität von Parsol 1798*)ohne Stabilisator | Photostabilität von Parsol 1789 mit 1% Stabilisator | Photostabilität von Parsol 1789 mit 2% Stabilisator |
|---|---|---|---|
| Verbindung aus Beispiel 1 | 30 min**): 64%<br>60 min: 28%<br>90 min: 12% | 30 min: 92%<br>60 min: 81%<br>90 min: 69% | 30 min: 94%<br>60 min: 90%<br>90 min: 85% |
| Verbindung aus Beispiel 2 | 30 min: 64%<br>60 min: 28%<br>90 min: 12% | 30 min: 95%<br>60 min: 81%<br>90 min: 73% | 30 min: 94%<br>60 min: 89%<br>90 min: 85% |
| Verbindung aus Beispiel 3 | 30 min: 64%<br>60 min: 28%<br>90 min: 12% | 30 min: 87%<br>60 min: 80%<br>90 min: 65% | 30 min: 96%<br>60 min: 85%<br>90 min: 81% |
| | | | |

*) 4-(1,1-Dimethylethyl)-4'-methoxydibenzoylmethan

**) Bestrahlungszeit

**Patentansprüche**

1. Kosmetische oder dermatologische Zubereitungen, enthaltend

a) 0,1 bis 10 Gew.-% eines oder mehrerer 3,3-Diphenylacrylate der Formel I,

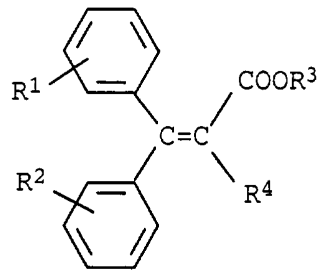

I

in der die Substituenten unabhängig voneinander folgende Bedeutung haben:

| | |
|---|---|
| $R^1$ und $R^2$ | Wasserstoff, $C_1$-$C_{12}$-Alkyl, $C_1$-$C_{12}$-Alkoxy; |
| $R^3$ | $C_3$-$C_{10}$-Cycloalkyl, gegebenenfalls substituiert; |
| $R^4$ | Wasserstoff, CN<br>und |

b) 0,1 bis 10 Gew.-% eines oder mehrerer Dibenzoylmethanderivate der Formel II,

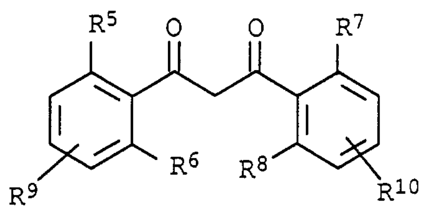

II

in der die Substituenten unabhängig voneinander folgende Bedeutung haben:

| | |
|---|---|
| $R^5$ bis $R^8$ | Wasserstoff, $C_1$-$C_4$-Alkyl; |
| $R^9$ und $R^{10}$ | Wasserstoff, $C_1$-$C_{12}$-Alkyl, $C_1$-$C_{12}$-Alkoxy. |

**2.** Kosmetische oder dermatologische Zubereitungen nach Anspruch 1, enthaltend

a) 0,1 bis 10 Gew.-% eines oder mehrerer 3,3-Diphenylacrylate der Formel Ib,

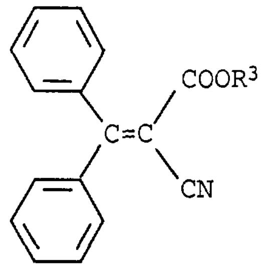

Ib

in der $R^3$ für $C_5$-$C_8$-Cycloalkyl, gegebenenfalls substituiert steht und

b) 0,1 bis 10 Gew.-% eines oder mehrerer Dibenzoylmethanderivate, ausgewählt aus der Gruppe, bestehend aus 2-Methyldibenzoylmethan, 4-Methyldibenzoylmethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4-tert.-Butyl-4'-methoxydibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan, 2,6-Dimethyl-4-tert.-butyl-4'-methoxydibenzoylmethan.

**3.** Kosmetische oder dermatologische Zubereitungen nach einem der Ansprüche 1 oder 2, enthaltend

a) 0,1 bis 10 Gew.-% eines oder mehrerer 3,3-Diphenylacrylate der Formel Ib,

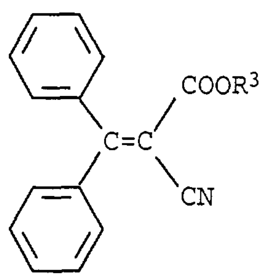

Ib

in der $R^3$ für $C_5$-$C_8$-Cycloalkyl, gegebenenfalls substituiert steht und

b) 0,1 bis 10 Gew.-% 4-tert.-Butyl-4'-methoxydibenzoylmethan.

**4.** Kosmetische oder dermatologische Zubereitungen nach einem der Ansprüche 1 bis 3, enthaltend 0,1 bis 6 Gew.-% der Komponente a).

**5.** Kosmetische oder dermatologische Zubereitungen nach einem der Ansprüche 1 bis 4, enthaltend 1,5 bis 2,5 Gew.-% der Komponente a).

**6.** Kosmetische oder dermatologische Zubereitungen nach einem der Ansprüche 1 bis 5, enthaltend 0,2 bis 7 Gew.-% der Komponente b).

7. Kosmetische oder dermatologische Zubereitungen nach einem der Ansprüche 1 bis 6, enthaltend 0,4 bis 5 Gew.-% der Komponente b).

8. Kosmetische oder dermatologische Zubereitungen nach einem der Ansprüche 1 bis 7, enthaltend die Komponenten a) und b) in einem molaren Verhältnis a:b von 0,5 bis 10.

9. Kosmetische oder dermatologische Zubereitungen nach einem der Ansprüche 1 bis 8, enthaltend die Komponenten a) und b) in einem molaren Verhältnis a:b von 1 bis 8.

10. Kosmetische oder dermatologische Zubereitungen nach einem der Ansprüche 1 bis 9 mit einem Lichtschutzfaktor > 10.

11. Verwendung von kosmetischen oder dermatologischen Zubereitungen, definiert gemäß Anspruch 1, zum Schutz der menschlichen Haut oder menschlichen Haare gegen Sonnenstrahlen, allein oder zusammen mit an sich für kosmetische und dermatologische Zubereitungen bekannten, im UV-Bereich absorbierenden Verbindungen.

Europäisches Patentamt

# EUROPÄISCHER RECHERCHENBERICHT

Nummer der Anmeldung
EP 01 10 1426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | US 5 587 150 A (DEFLANDRE ANDRE ET AL) 24. Dezember 1996 (1996-12-24)<br>* Zusammenfassung *<br>* Spalte 1, Zeile 5 - Zeile 17 *<br>* Spalte 1, Zeile 47 - Spalte 2, Zeile 54 *<br>* Spalte 3, Zeile 1 - Zeile 24 *<br>* Beispiele *<br>* Ansprüche 1-6,16-23,31-33 *<br>--- | 1-11 | A61K7/42 |
| A | US 5 989 528 A (IRWIN CHRISTOPHER ET AL) 23. November 1999 (1999-11-23)<br>* Zusammenfassung *<br>* Spalte 2, Zeile 21 - Zeile 25 *<br>* Spalte 2, Zeile 48 - Spalte 3, Zeile 32 *<br>* Spalte 4, Zeile 14 - Spalte 5, Zeile 42 *<br>* Ansprüche *<br>--- | 1-8,10, 11 | |
| A | EP 0 780 119 A (GIVAUDAN ROURE INT) 25. Juni 1997 (1997-06-25)<br>* Zusammenfassung *<br>* Seite 2, Zeile 1 - Zeile 27 *<br>* Seite 2, Zeile 57 - Seite 3, Zeile 26 *<br>* Beispiele *<br>* Ansprüche *<br>--- | 1-8,10, 11 | RECHERCHIERTE SACHGEBIETE (Int.Cl.7)<br>A61K |
| A | US 5 280 380 A (BYKER HARLAN J) 18. Januar 1994 (1994-01-18)<br>* Zusammenfassung *<br>* Spalte 1, Zeile 9 - Zeile 12 *<br>* Spalte 1, Zeile 50 - Zeile 68 *<br>* Spalte 2, Zeile 39 - Zeile 44 *<br>* Spalte 3, Zeile 20 - Zeile 59 *<br>---<br>-/-- | | |

Der vorliegende Recherchenbericht wurde für alle Patentansprüche erstellt

| Recherchenort | Abschlußdatum der Recherche | Prüfer |
|---|---|---|
| DEN HAAG | 3. Juli 2001 | Cielen, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

Europäisches Patentamt

# EUROPÄISCHER RECHERCHENBERICHT

Nummer der Anmeldung
EP 01 10 1426

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 3 275 520 A (GEN ANILINE & FILM CORP) 27. September 1966 (1966-09-27)<br>* Spalte 1, Zeile 1 - Spalte 5, Zeile 5 *<br>* Spalte 12, Zeile 37 - Zeile 39 *<br>* Beispiel 6 *<br>* Anspruch 1 * | | |
| A | GB 976 968 A (GEN ANILINE & FILM CORP) 2. Dezember 1964 (1964-12-02)<br>* Seite 1, Zeile 11 - Zeile 27 *<br>* Seite 2, Zeile 16 - Zeile 44 *<br>* Seite 2, Zeile 60 - Seite 3, Zeile 4 *<br>* Beispiel 8 *<br>* Seite 7, Zeile 87 - Zeile 105 *<br>* Ansprüche 1-5 * | | |
| A | DATABASE WPI<br>Week 198427<br>Derwent Publications Ltd., London, GB;<br>AN 1984-170264<br>XP002171070<br>PANKOVA T.A.,PARAMONOV V.I.,POPOVA Z.G.: "Process for preparing 2,2,6,6-tetramethyl-4-piperidyl-beta,beta-prim-diphenyl-alpha-cyaneacrylate"<br>& SU 1 051 075 A (POLYM MAT CHEMICALS), 30. Oktober 1983 (1983-10-30)<br>* Zusammenfassung * | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 3. Juli 2001 | Cielen, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 01 10 1426

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-07-2001

| Im Recherchenbericht angeführtes Patentdokument | Datum der Veröffentlichung | Mitglied(er) der Patentfamilie | Datum der Veröffentlichung |
|---|---|---|---|
| US 5587150 A | 24-12-1996 | FR 2658075 A | 16-08-1991 |
| | | AT 96654 T | 15-11-1993 |
| | | AU 652742 B | 08-09-1994 |
| | | AU 7310591 A | 03-09-1991 |
| | | CA 2076003 A,C | 15-08-1991 |
| | | DE 69100593 D | 09-12-1993 |
| | | DE 69100593 T | 31-03-1994 |
| | | DK 514491 T | 29-11-1993 |
| | | EP 0514491 A | 25-11-1992 |
| | | ES 2060370 T | 16-11-1994 |
| | | WO 9111989 A | 22-08-1991 |
| | | JP 2975682 B | 10-11-1999 |
| | | JP 5504572 T | 15-07-1993 |
| | | PT 96709 A,B | 31-10-1991 |
| | | US 5576354 A | 19-11-1996 |
| | | ZA 9101104 A | 27-11-1991 |
| US 5989528 A | 23-11-1999 | AU 5240699 A | 21-02-2000 |
| | | BR 9912036 A | 03-04-2001 |
| | | EP 1100447 A | 23-05-2001 |
| | | WO 0006110 A | 10-02-2000 |
| EP 0780119 A | 25-06-1997 | AU 719298 B | 04-05-2000 |
| | | AU 7540396 A | 26-06-1997 |
| | | IL 119814 A | 16-07-2000 |
| | | JP 9175974 A | 08-07-1997 |
| | | US 6033649 A | 07-03-2000 |
| US 5280380 A | 18-01-1994 | US 5148305 A | 15-09-1992 |
| US 3275520 A | 27-09-1966 | KEINE | |
| GB 976968 A | 02-12-1964 | KEINE | |
| SU 1051075 A | 30-10-1983 | KEINE | |

EPO FORM P0461

Für nähere Einzelheiten zu diesem Anhang : siehe Amtsblatt des Europäischen Patentamts, Nr.12/82